# EUROPEAN PATENT SPECIFICATION

(11) **EP 2 508 156 B1**
(45) Date of publication and mention of the grant of the patent: **31.12.2014**
(21) Application number: 10834514.1
(22) Date of filing: 24.11.2010
(51) Int. Cl.: A61F 13/15, A61F 13/49

(54) **METHOD OF MANUFACTURING ABSORBENT MEMBER AND APPARATUS USED TO MANUFACTURE SAME**
VERFAHREN ZUR HERSTELLUNG EINES ABSORBIERENDEN ELEMENTES UND BENUTZTE VORRICHTUNG ZUR HERSTELLUNG DESSELBEN
PROCÉDÉ DE FABRICATION DE CORPS D'ABSORPTION ET DISPOSITIF EMPLOYÉ DE FABRICATION DE CELUI-CI

(30) Priority: 04.12.2009 JP 2009276015; 22.12.2009 JP 2009291522
(43) Date of publication of application: 10.10.2012
(73) Proprietor: Kao Corporation, Chuo-Ku Tokyo 103-8210 (JP)
(72) Inventor: MOTEGI, Tomoyuki, Haga-gun Tochigi 321-3497 (JP); MARUYAMA, Hiroshi, Haga-gun Tochigi 321-3497 (JP); ONIZAWA, Yasuhiro, Haga-gun Tochigi 321-3497 (JP); MORITA, Akio, Haga-gun Tochigi 321-3497 (JP)
(74) Representative: Vossius & Partner
(86) International application number: PCT/JP2010/070911
(87) International publication number: WO 2011/068062

(56) References cited:
- EP-A1- 1 154 051
- EP-A1- 1 621 166
- WO-A2-2004/011723
- JP-A- 4 161 153
- JP-A- 7 010 239
- JP-A- 2005 304 815
- JP-A- 2006 016 727
- JP-A- 2006 115 911
- JP-A- 2006 141 615
- JP-A- 2008 206 539
- JP-A- 2008 508 036
- JP-A- 2009 072 421

## Description

### Technical Field

This invention relates to a method of manufacturing an absorbent member and apparatus used to manufacture the same.

### Background Art

In a method of manufacturing an absorbent member used for absorbent articles such as disposable diaper, sanitary napkin and incontinent pad, it is well known to accumulate material for the absorbent member (fibrous material such as fluff pulp) supplied by air flow in the recesses formed on the outer peripheral surface of the rotary drum under a suction effect and to release the accumulations molded in the shapes corresponding to those of the recesses onto a transfer roll or a vacuum conveyor (See, for example, WO2005/072671A1).

It is also well known to accumulate material for the absorbent member fed on the air flow in the recesses formed on the outer peripheral surface of the rotary drum under a suction effect, to transfer accumulations molded in the shapes corresponding to those of the recesses onto a transfer roll, to convey the accumulations while the accumulations being retained on the transfer roll and to transfer the accumulations onto the other conveying means such as a conveyor to the subsequent step (See, for example, JP2002-272782A and JP2006-115911A).

According to the prior art, the accumulations having been released in this manner are used as the absorbent member directly or after combined with the other sheet material. According to the conventional method to release the accumulations from the recesses of the rotary drum, the air is jetted out from the bottoms of the recesses, on one hand, and the accumulations are subjected to a suction force from the side of the transfer roll on which the accumulations are to be transferred, on the other hand.

WO 2004/001723 A2 discloses a rotary drum having a forming surface with a series of form members. Vacuum is used to suck fibers into the form members to form individual absorbent cores. In a release zone, vacuum causes the accumulated fibrous web to transfer from the forming surface onto a conveyor. This is done by using vacuum suction box.

### Summary of Invention

### Technical Problem

However, the inventors used a following method in order to manufacture a absorbent member in which a plurality of accumulations (molded product of the material) are arranged in a predetermined pattern. In the method, a rotary drum having, on its outer peripheral surface, a plurality of recesses arranged in the circumferential and the width directions for accumulating material was used. The material was accumulated in the recesses by sucking to obtain a plurality of accumulations. Then, the accumulations were transferred from the recesses to a transfer roll or a vacuum conveyor and were conveyed while being retained on the transfer roll or the vacuum conveyor. In this method, however, the inventors found that, in the course of transfer or during conveyance, the accumulations located in the vicinity of the opposite side edges of the conveying means as viewed in the direction orthogonal to the conveying direction sometimes slumped against the accumulations located in the vicinity of the central zone and, in addition, the positional relationship among the accumulations was sometimes disturbed. Occurrence of such situation is undesirable from the viewpoint of manufacturing the absorbent member consisting of a plurality of the accumulations (molded products of the material) orderly arranged in the predetermined pattern at a high yield grade and from the viewpoint of assuring the flexibility and absorptive property of the absorbent member.

Furthermore, according to the conventional method, there was possibility that the accumulations might get out shapes and/or the positional relationship among the accumulations might be disturbed due to the air flow in the course of transferring the accumulations onto the transfer roll or the like depending on the shape, size and arrangement of the recesses formed on the rotary drum.

According to the disclosure of WO2005/072671A1, between the region in which the material is fed to the outer peripheral surface of the rotary drum (rotary molding wheel) and the position at which the accumulation from the rotary molding wheel to the transfer roll, the air-permeable belt are supplied onto the outer peripheral surface of the rotary drum and at the same time the accumulations are subjected to a suction force provided from the inside of the rotary drum to prevent drop off of the particles from the accumulations before the accumulations are transferred onto the transfer roll or the like. Even in this case, there is still the possibility that the accumulations might get out shapes and/or the positional relationship among the accumulations might be disturbed.

### Solution to Problem

This invention provides a method for manufacturing an absorbent member including an accumulating step including accumulating material for the absorbent member carried by air flow in recesses formed on an outer peripheral surface of a rotary drum by sucking, and a conveying step including transferring accumulations in the recesses onto conveying means and conveying the accumulations while the accumulations being retained on the conveying means. In the accumulating step, the material for the absorbent member is accumulated in a plurality of the recesses formed on the rotary drum so as to be arranged in a width direction of the rotary drum. In the conveying step, the accumulations are released from the recesses onto the conveying means under a suction force provided from the conveying means and the released accumulations are conveyed while being sucked.

This invention provides also an apparatus for manufacturing the absorbent member including a rotary drum having recesses on its outer peripheral surface which have a plurality of fine pores on respective bottoms. The apparatus is configured to accumulate material for the absorbent member supplied from a material feeding zone in the respective recesses, to release these accumulations from the recesses and to transfer them onto conveying means so that the accumulations may be conveyed while being retained on the conveying means. A plurality of the recesses are arranged so as to be spaced from each other in a circumferential direction as well as in a width direction of the rotary drum.

### Advantageous Effects of Invention

With use of the method and the apparatus according to this invention for manufacturing the absorbent member, it is possible to prevent the accumulations (molded products of the material) obtained by accumulating the material within the recesses under a suction effect from turning over or getting out of shapes or position-disturbed in the course of being transferred onto the conveying means or in the course of being conveyed. In this way, the absorbent member consisting of a plurality of the accumulations (molded products of the material) arranged in a predetermined pattern can be efficiently manufactured.

With use of the method and the apparatus according to this invention for manufacturing the absorbent member, there is almost no anxiety that the accumulations might get out of shapes of the position relationship among the accumulations might be disturbed in the course of transferring the accumulations from the recesses of the rotary drum to the other conveying means and the desired absorbent members can be efficiently manufactured.

### Brief Description of Drawings

[Fig.1] Fig. 1 is a diagram schematically illustrating an apparatus according to a first embodiment of this invention used to manufacture absorbent members.
[Fig. 2] Fig. 2 is an enlarged diagram schematically illustrating a transfer roll and vicinity thereof in the apparatus illustrated in Fig. 1.
[Fig. 3] Fig. 3 (a) is a developed view partially illustrating an outer peripheral surface of a rotary drum in the apparatus illustrated in Fig. 1 and Fig. 3(b) is a sectional view of the outer peripheral surface of the rotary drum.
[Fig. 4] Fig. 4 is a developed view partially illustrating an outer peripheral surface of the transfer roll in the apparatus illustrated in Fig. 1.
[Fig. 5] Fig. 5 is a view illustrating a windshield plate in the apparatus illustrated in Fig. 1 as viewed in a direction of an arrow A in Fig. 2.
[Fig. 6] Fig. 6 is a perspective view of the transfer roll and the windshield plate in the apparatus illustrated in Fig. 1.
[Fig. 7] Fig. 7 exemplarily illustrates an absorbent member manufactured by the apparatus illustrated in Fig. 1 wherein Fig. 7 (a) is a partially cutaway plan view and Fig. 7 (b) is a schematic sectional view taken along a line B-B in Fig. 7 (a).
[Fig. 8] Fig. 8 is a diagram exemplarily illustrating inter-column partition plates arranged between the windshield plates in an apparatus according to a second embodiment of this invention for manufacturing of the absorbent member (diagram corresponding to Fig. 5).
[Fig. 9] Fig. 9 is a perspective view exemplarily showing inter-column partition plates arranged between the windshield plates in the apparatus according to a second embodiment of this invention for manufacturing of the absorbent member (diagram corresponding to Fig. 6).
[Fig. 10] Fig. 10 is a diagram schematically illustrating an apparatus according to a third embodiment of this invention for manufacturing of the absorbent member.
[Fig. 11] Fig. 11 is a perspective diagram schematically illustrating the absorbent member manufactured by the apparatus illustrated in Fig. 10.
[Fig. 12] Fig. 12 (a) is a sectional view exemplarily showing recesses formed on the outer peripheral surface of the rotary drum and Fig. 12 (b) is a sectional view exemplarily showing the absorbent member manufactured with use of the rotary drum shown in Fig. 12 (a).
[Fig. 13] Fig. 13 is a perspective view showing another example of the absorbent member manufactured according to this invention.
[Fig. 14] Fig. 14 is a diagram schematically illustrating an apparatus according to a fourth embodiment of this invention used to manufacture the absorbent members.
[Fig. 15] Fig. 15 (a) and Fig. 15(b) exemplarily illustrating the recesses formed on the outer peripheral surface of the rotary drum in the apparatus illustrated in Fig. 14 wherein Fig. 15 (a) is a diagram illustrating the outer peripheral surface of the rotary drum as viewed from above in a normal direction and Fig. 15 (b) is a schematic sectional diagram taken along a line F-F in Fig. 15(a).
[Fig. 16] Fig. 16 is a perspective view showing the transfer roll, a vacuum box and vicinity thereof in the apparatus illustrated in Fig. 14.
[Fig. 17] Fig. 17 is a schematic sectional diagram illustrating a state of accumulations within the respective recesses in the course of being conveyed under the effect of suction provided from the side opposite to the rotary drum.
[Fig. 18] Fig. 18 (a) and Fig. 18 (b) are diagrams exemplarily illustrating the absorbent member manufactured by the apparatus illustrated in Fig. 14 wherein Fig. 18 (a) is a partially cutaway plan diagram and Fig. 18 (b) is a schematic sectional diagram taken along a line G-G in Fig. 18 (a).

### Description of Embodiments

This invention will be described on the basis of preferred embodiments thereof in reference to the accompanying drawings.

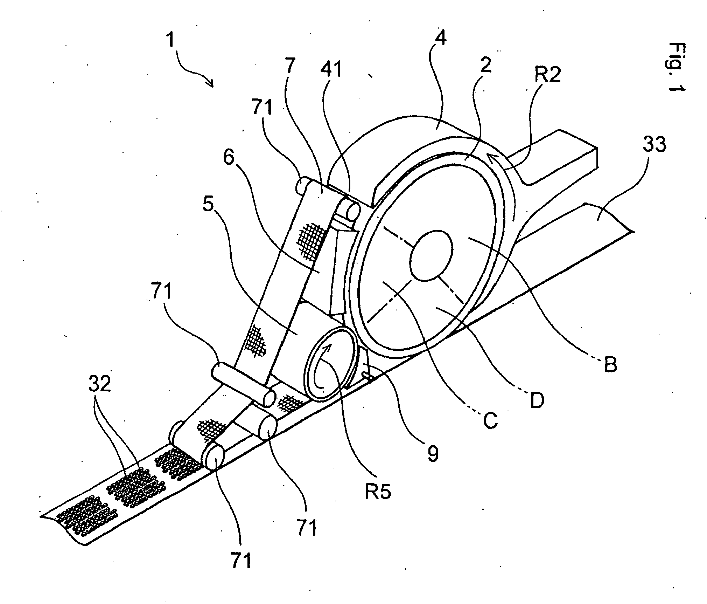

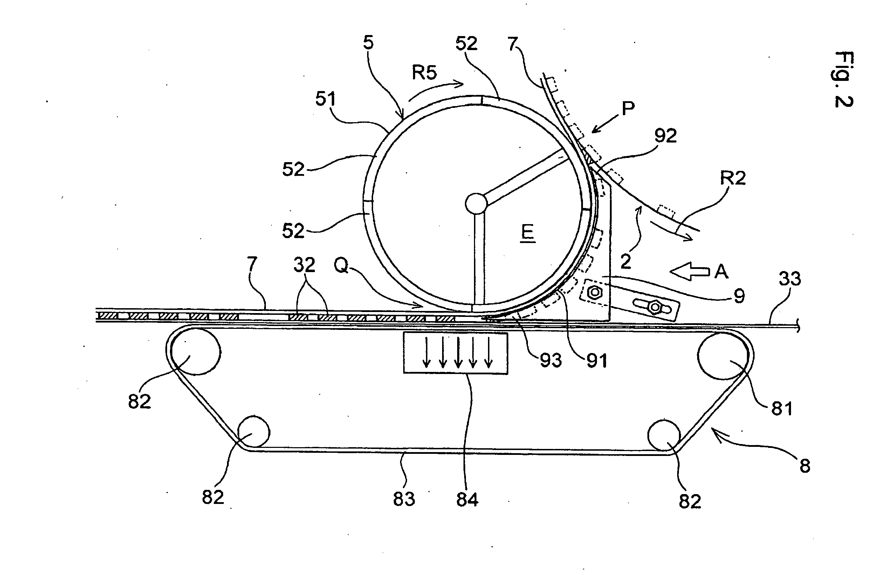

An apparatus 1 according to a first embodiment of this invention used to manufacture an absorbent member includes, as illustrated in Figs. 1 and 2, a rotary drum 2 adapted to be rotationally driven in a direction of an arrow R2, a duct 4 serving to feed fibrous material as basic ingredient of the absorbent member onto an outer peripheral surface of the rotary drum 2, a transfer roll 5 set up obliquely below the rotary drum 2 and adapted to be rotationally driven in a direction of an arrow R5, a vacuum box 6 set up between the duct 4 and the transfer roll 5 as viewed in the peripheral direction of the rotary drum 2, mesh belt 7 set up so as to pass between the vacuum box 6 and the rotary drum 2 and between the transfer roll 5 and the rotary drum 2, vacuum conveyor 8 set up below the transfer roll 5 and windbreak plates 9 shaped so as to follow a curve of the outer peripheral surface of the transfer roll 5 and set up close to the mesh belt 7.

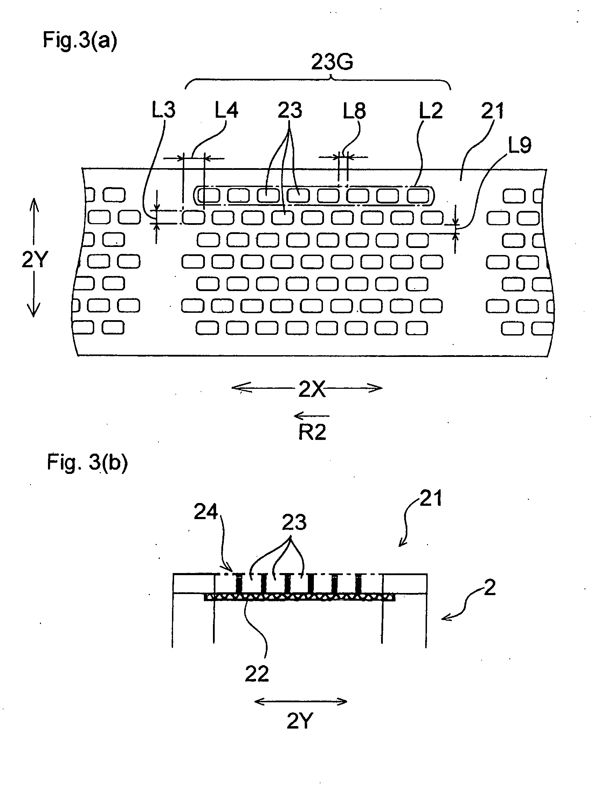

As illustrated in Fig. 1, the rotary drum 2 has a cylindrical shape and is driven by a power engine such as electric motor to be rotated around a horizontal axis. As illustrated in Fig. 3, the rotary drum 2 has a plurality of recesses 23 on its outer peripheral surface 21 which have a bottom formed of a mesh plate 22. In Fig. 3, a direction 2X indicates a circumferential direction of the rotary drum 2 and a direction 2Y indicates a width direction of the outer peripheral surface of the rotary drum 2 (i.e., a direction in parallel to an axis of rotation of the rotary drum 2).

A plurality of the recesses 23 of the rotary drum 2 are spaced from each other, as illustrated in Fig. 3, in the circumferential direction (direction 2X) as well as in the width direction (direction 2Y).

More specifically, a plurality of columns L2 each including a plurality of the recesses 23 serially arranged in the circumferential direction (direction 2X) of the outer peripheral surface of the rotary drum 2 are formed in the width direction (direction 2Y) of the rotary drum 2 and, between each pair of the adjacent recess columns L2, a region having none of the recesses 23 continuously extends in the direction 2X.

A plurality of the recesses 23 are really formed by fixing a patterned plate 24 formed with a plurality of through pores each having a same cross-sectional shape as that of the recess 23 to the outer peripheral surface of the rotary drum 2 so that the patterned plate 24 may be lapped on the mesh plate 22. An inner peripheral surface of the recess 23 is defined by an inner peripheral surface of the through-pore of the patterned plate 24. The mesh plate 22 has a plurality of sufficiently fine pores to block the material for the absorbent member carried by the air flow but to allow passage of only the air through these fine pores.

An irrotational portion defined inside the rotary drum 2 (on the side of the axis of rotation) is formed with a space B adapted to be depressurized. An air exhaust system of well known art such as an intake fun (not shown) is connected to this space B so that the space B may be maintained at a negative pressure by actuating such air exhaust system.

Also inside the rotary drum 2 (on the side of the axis of rotation), a space C is defined and air flows from the outside into this space C under the suction force of provided from the side of the vacuum box 6 to be described later on, and a space D is defined and air flows into the space D under the suction force provided from the side of the transfer roll 5. The respective spaces are partitioned by plates extending inside the rotary drum toward the axis of rotation.

As illustrated in Fig. 1, the segment of the duct 4 on the side of one end thereof covers the segment of the outer peripheral surface of the rotary drum 2 defined above the space B and the segment of the duct 4 on the side of the other end thereof not illustrated in Fig. 1 includes therein a fibrous material delivery device. The fibrous material delivery device includes, for example, a grinder mill adapted to crush sheet-like wood pulp into fluff pulp (fibrous material) to be delivered into the duct. It is possible to set up a liquid-absorbent polymer delivery device along the duct 4.

During passage of the individual recesses 23 of the rotary drum 2 above the space B maintained at a negative pressure, a suction force is provided through the fine pores formed in the bottoms of the recesses 23. Such suction force generates air flow serving to feed the material for the absorbent member delivered from the fibrous material delivery device and the water-absorbent polymer delivery device having been delivered into the duct 4 to the outer peripheral surface of the rotary drum 2. The materials fed by the air flow in this manner are accumulated in the recesses 23.

The transfer roll 5 has an air- permeable cylindrical outer periphery and is driven by a power engine such as an electric motor to be rotated around a horizontal axis.

An irrotational portion defined inside the transfer roll 5 (on the side of its axis of rotation) is formed with a space E adapted to be depressurized. An air exhaust system of well known art such as an air intake fun (not shown) is connected to this space E so that the space E may be maintained at a negative pressure by actuating such air exhaust system.

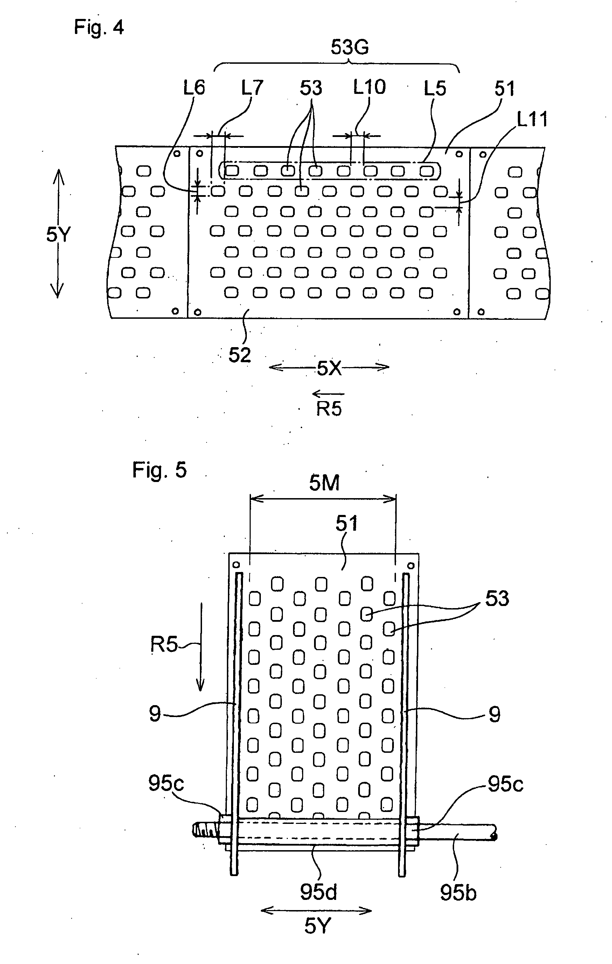

As illustrated in Fig. 4, the transfer roll 5 is formed on its outer peripheral surface 51 with a plurality of suction pores 53. In Fig. 4, a direction 5X indicates a circumferential direction of the outer peripheral surface of the transfer roll 5 and a direction 5Y indicates a width direction of the outer peripheral surface 51 of the transfer roll 5 (i.e., a direction in parallel to an axis of rotation of the transfer roll 5).

A plurality of the suction pores 53 of the transfer roll 5 are formed so as to be spaced from each other, as illustrated in Fig. 4, in the circumferential direction (direction 5X) as well as in the width direction (direction 5Y) of the transfer roll 5. More specifically, a plurality of columns L5 of suction pores each including a plurality of the suction pores 53 serially arranged in the circumferential direction (direction 5X) of the transfer roll 5 are formed in the width direction (direction 5Y) of the transfer roll 5 and, between each pair of the adjacent columns L5 of the suction pores, a region having none of the suction pores 53 continuously extends in the direction 5X.

A plurality of the suction pores 53 are really formed by fixing a plate 52 formed with a plurality of through pores each having a same cross-sectional shape as that of the suction pore 53 to the outer periphery of the transfer roll 5. During passage above the space E maintained at a negative pressure, the individual suction pores 53 functions to suck the ambient air.

The suction pores 53 of the transfer roll 5 are arranged so as to be associated with the recesses 23 of the rotary drum 2 so that accumulations 32 of the material in the recesses 23 of the rotary drum 2 may be individually subjected to the suction force provided from the associated suction pores 53 and transferred onto the transfer roll 5.

More specifically, both a center point placement pattern and a center-to-center distance of the recesses 23 of the rotary drum 2 are same as those of the suction pores 53 of the transfer roll 5. The rotary drum 2 and the transfer roll 5 are synchronously rotated in the same phase so that the individual suction pores 53 may overlap the individual associated recesses 23. In this way, the individual recesses 23 are subjected to the suction force provided by the individual associated suction pores 53.

In the apparatus 1 according to this embodiment, the rotary drum 2 includes a plurality of recess groups 23G, each consisting of a plurality of the recesses 23 used to form the accumulations 32 to be included in each of the individual absorbent articles, and arranged so to be spaced each other in the circumferential direction of the rotary drum 2. In a similar way, the transfer roll 5 includes a plurality of suction pore groups 53G, each consisting of a plurality of the suction pores 53 used to subject the accumulations 32 to be included in each of the individual absorbent articles to the suction force, and arranged so as to be spaced each other in the circumferential direction of the transfer roll 5. Though not specified, the number of the recess groups 23G formed on the outer peripheral surface of the rotary drum 2 may be, for example, in a range of 2 to 20 and the number of the suction pore groups 53G formed on the outer peripheral surface 51 of the transfer roll 5 may be, for example, in a range of 1 to 10.

The vacuum box 6 is set up between a downstream end 41 of the duct 4 and the transfer roll 5 as viewed in the rotational direction of the rotary drum 2.

As illustrated in Fig. 1, the vacuum box 6 has a box-like configuration defined by upper and lower surfaces, opposite side surfaces and a rear surface wherein a region opposed to the rear surface is provided with an opening which opens toward the rotary drum 2.

The vacuum box 6 is connected to an air exhaust system (not shown) of well known art such as an air intake fan via an exhaust pipe or the like (not shown) so that the interior of the vacuum box 6 may be maintained at a negative pressure by actuation of the air exhaust system.

The mesh belt 7 is belt-like air-permeable mesh belt provided in the form of an endless belt and guided a plurality of free rolls 71 and the transfer roll 5 to run continuously along a predetermined pathway. The mesh belt 7 is driven by rotation of the transfer roll 5. As illustrated in Fig. 1, the mesh belt 7 is set up so as to be guided onto the outer peripheral surface of the rotary drum 2 in the vicinity of the downstream side end 41 of the duct 4, then sequentially to pass between the vacuum box 6 and the rotary drum 2 and between the transfer roll 5 and the rotary drum 2.

The mesh belt 7 is kept in contact with the outer peripheral surface of the rotary drum 2 during passage of the mesh belt 7 in front of the opening of the vacuum box 6 and, in the vicinity of a region in which the transfer roll 5 and the rotary drum 2 come closest to each other, the mesh belt 7 is transferred from the outer peripheral surface of the rotary drum 2 onto the transfer roll 5.

The mesh belt 7 has meshes (fine pore) remarkably smaller than the suction pores 53 formed through the outer peripheral surface 51 of the transfer roll 5. Regions of the mesh belt 7 overlapping the suction pores 53 are formed with suction regions each having a planar shape same as that of the associated suction pore 53 so that the associated accumulation released from the recess may stick to the associated suction region.

"Conveying unit" in this embodiment consists of the transfer roll 5 having the suction pores 53 and the mesh belt 7 adapted to run in contact with the outer peripheral surface 51 of the transfer roll 5. The suction pores in this embodiment consist of the suction pores 53 formed through the outer peripheral surface 51 of the transfer roll 5 or these suction pores 53 and the regions of the mesh belt 7 respectively overlapping these suction pores 53 (the aforementioned suction regions).

A mesh open area ratio in the mesh belt 7 is preferably in a range of 10 to 90% and more preferably in a range of 40 to 60%.

A mesh size in the mesh belt 7 is preferably in a range of Ø 0.1mm to Ø 3.0mm and more preferably in a range of Ø 1.0mm to Ø 2.0mm.

Material for the mesh belt may be selected from various kinds of well known material but the mesh belt is preferably made from resinous material.

The vacuum conveyor 8 includes an endless air-permeable belt 83 running along a driving roll 81 and driven rolls 82, 82 and a vacuum box 84 set up at a location opposed to the transfer roll 5 across the air-permeable belt 83.

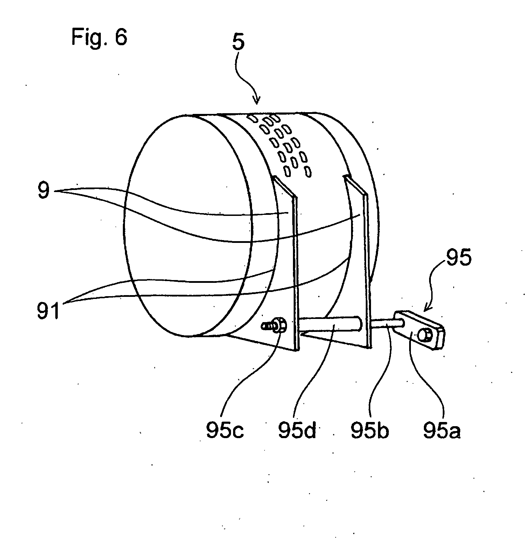

As illustrated in Figs. 5 and 6, the windbreak plate 9 is set up so as to be paired on both sides of a region 5M of the outer peripheral surface 51 of the transfer roll 5 defined in the width direction (direction 5Y) in which the suction pores 53 are formed. The windbreak plates 9 are set up substantially perpendicularly to the outer peripheral surface 51 of the transfer roll 5.

As illustrated in Fig. 2, each of the windbreak plates 9 has a circular-arc edge 91 shaped so as to follow the surface of the mesh belt 7 extending along the outer peripheral surface 51 of the transfer roll 5 and set up close to the surface of the mesh belt 7. Each of the windbreak plates 9 has first corner 92 tapered toward a region in which the rotary drum 2 comes closest to the transfer roll 5 and a second corner 93 tapered toward a region in which the transfer roll 5 comes closest to the air-permeable belt 83 of the vacuum conveyor 8 to reduce an amount of air which would otherwise flow from the lateral side over a range as wide as possible, i.e., the range extending from the region in which the rotary drum 2 comes closest to the transfer roll 5 to the range in which the transfer roll 5 comes closest to the air-permeable belt 83 of the vacuum conveyor 8. These windbreak plates 9 preferably have a sufficient height to define walls in both side of regions sandwiching the region 5M which are higher than a height of the accumulations 32 sticking to the outer peripheral surface of the transfer roll 5.

A clearance (distance) between the circular-arc-shaped edge 91 of the windbreak plate 9 and the outer peripheral surface 51 of the transfer roll 5 (or the surface of the mesh belt 7 when the mesh belt 7 is transported onto the outer peripheral surface 51 of the transfer roll 5 as in this embodiment) is preferably 10mm or less and more preferably 1mm or less from the viewpoint of preventing, for example, the accumulation from slumping due to the air flowing in sideways.

While either type or kind of support means for the windbreak plates 9 is not limited, the support means 95 used in the embodiment illustrated in Figs. 5 and 6 includes a stationary portion 95a fixed to wall surface, a rod-like member 95b planted on the stationary portion 95a, nuts 95c, 95c screwed together with external thread of the rod-like member 95b and a cylindrical spacer 95d.

Now a method of manufacturing the absorbent members 3 in a continuous manner with use of the aforementioned manufacturing apparatus 1 for the absorbent members 3, i.e., a manufacturing method according to this invention for the absorbent member will be described hereunder on the basis of one embodiment thereof.

To manufacture the absorbent members 3 with use of the absorbent member manufacturing apparatus 1, the exhaust systems respectively connected to the space B within the rotary drum 2, the space E within the transfer roll 5 and the space within the vacuum box 6 are actuated to maintain these spaces at negative pressure. The negative pressure maintained within the space B generates an air flow within the duct 4 and this air flow functions to convey the material for the absorbent members toward the outer peripheral surface of the rotary drum 2. The rotary drum 2 and the transfer roll 5 are synchronously rotated so that the aforementioned recess groups 23G and the suction pore groups 53G may overlap each other and the individual recesses 23 in these recess groups 23G and the associated individual suction pores 53 in the suction pore groups 53G may overlap each other. The vacuum conveyor 8 also is actuated.

When the fibrous material delivery device is actuated to feed the fibrous material into the duct 4, the fibrous material is scattered and borne on the air flow and fed toward the outer peripheral surface of the rotary drum 2.

In the course of being conveyed immediately under the duct 4, the fibrous material 31 is accumulated in the recesses 23 of the rotary drum 2 under the suction force. Thereupon, the accumulations 32 accumulated in the recesses 23 are shaped so as to follow the shape of the inner peripheral surface of the respective recesses 23.

When these recesses 23 come to a position opposed to the vacuum box 6 as the rotary drum 2 rotates, the accumulations 32 in the respective recesses 23 stick to the mesh belt 7 under the suction force provided from the vacuum box 6. The accumulations 32 within the respective recesses 23 are conveyed in such state to a position immediately near to the position P at which the transfer roll 5 and the rotary drum 2 come closest to each other.

In the vicinity of the position P at which the transfer roll 5 and the rotary drum 2 come closest to each other, the accumulations 32 within the respective recesses 23 are released from the recesses 23 under the suction force individually exerted through the associated suction pores 53 on the individual recesses 23 and transferred together with the mesh belt 7 onto the transfer roll 5.

The accumulations 32 transferred together with the mesh belt 7 onto the transfer roll 5 are conveyed still under the suction force through the suction pores 53 associated with the individual recesses 23 to a delivery point Q at which the accumulations 32 are transferred onto the vacuum conveyor 8 under the suction force provided from the vacuum box 84.

According to this embodiment, a first core wrapping sheet 33 such as tissue paper or liquid-permeable nonwoven fabric is introduced onto the vacuum conveyor 8 before the accumulations 32 are transferred onto the vacuum conveyor 8 and then the accumulations 32 are transferred onto this first core wrapping sheet 33 as illustrated in Figs. 1 and 2.

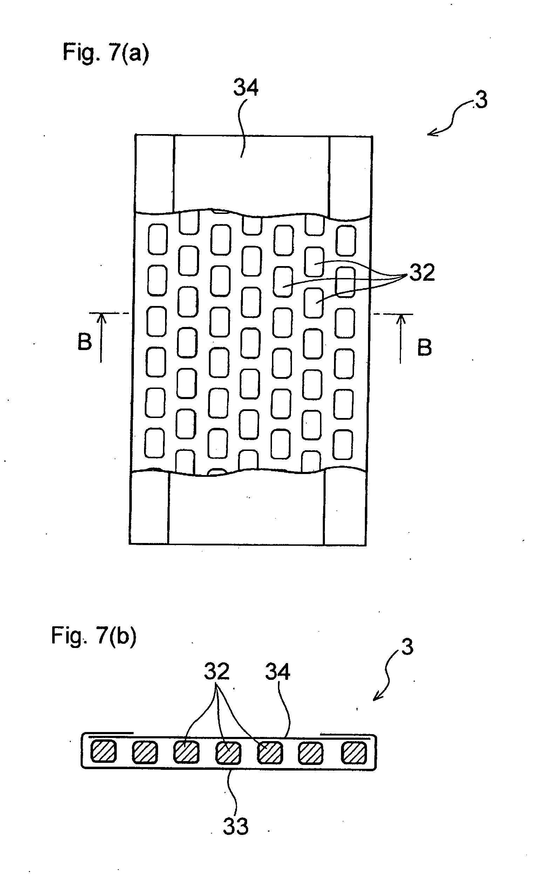

Then, at a downstream point, a second core wrapping sheet 34 is laminated on the accumulations 32 having bottoms already wrapped with the first wrapping sheet 33 and these accumulations 32 wrapped with the first and second wrapping sheets 33, 34 are cut at predetermined intervals corresponding to a dimension of the individual absorbent member to obtain the absorbent member 3 illustrated in Fig. 7.

While the first and second wrapping sheets 33, 34 may be bonded to each other without folding back these wrapping sheets 33, 34, opposite lateral portions of one of the sheets may be folded upward or downward to the side of the other wrapping sheet and then fixed together by means of adhesive or the other technique as in the case of the absorbent member illustrated in Fig. 7.

In the manufacturing method according to this embodiment, the respective accumulations 32 in a plurality of the recesses 23 of the rotary drum 2 are individually subjected to the suction force provided from the associated suction pores 53 so as to be transferred onto the mesh belt 7 on the transfer roll 5 and then these accumulations 32 released from a plurality of the recesses 23 are conveyed while being individually sucked. With such arrangement, a volume of the air (wind) flowing into the transfer roll 5 through the clearance among the accumulations 32 can be significantly reduced in comparison to the case in which the clearance among the accumulations 32 also are subjected to the suction force. Consequently, it is possible to prevent various problems such that, in the course of transfer to the conveyor means or during conveyance, the accumulations located in the vicinity of the opposite side edges of the roll 5 might slump against the accumulations located in the midsection of the roll's peripheral surface, the accumulations might loose their initial shape and/or a positional relationship of the accumulations might be disturbed.

In the manufacturing apparatus according to this embodiment, the many recesses 23 in which the material are accumulated are formed on the outer peripheral surface so as to be spaced from each other in the circumferential direction as well as in the width direction of the rotary drum 2, on one hand, and the outer peripheral surface of the transfer roll 5 is provided with the suction pores 53 adapted to subject the accumulations in the many recesses 23 to the suction force individually, on the other hand. In this way, it is possible for the suction pores 53 to suck the accumulations 32 released from the respective recesses 23 in the individual manner. Therefore, a volume of the air (wind) flowing into the transfer roll 5 through the clearance among the accumulations 32 can be significantly alleviated in comparison to the case in which the clearance among the accumulations 32 also are subjected to the suction force. Consequently, it is possible to prevent various problems such that, in the course of transfer to the conveyor means or during conveyance, the accumulations located in the vicinity of the opposite side edges of the roll 5 might slump against the accumulations located in the midsection of the roll's peripheral surface, the accumulations might loose their initial shape and/or a positional relationship of the accumulations might be disturbed.

In order to improve the releasability of the accumulations from the associated recesses, to prevent the accumulations from slumping and to prevent the positional relationship of the accumulations from being disturbed, a dimension L6 (See Fig. 4) of the individual suction pore 53 formed on the peripheral surface of the transfer roll 5 measured in the width direction of the transfer roll is preferably equal to or smaller than a dimension L3 (See Fig. 3) of the individual recess 23 formed on the peripheral surface of the rotary drum 2 measured in the width direction of the rotary drum 2 and a dimension L7 (See Fig. 4) of the suction pore 53 measured in the circumferential direction of the transfer roll 5 is preferably equal to or smaller than a dimension L4 (See Fig. 3) of the recess 23 measured in the circumferential direction (direction 2X) of the rotary drum 2.

A cross-sectional area of the individual suction pore 53 (cross-sectional area of the plane in parallel to the outer peripheral surface 51) is preferably equal to or smaller than the area of the opening of the recess 23, specifically, in a range of 20 to 100% and preferably in a range of 30 to 60% of the area of the opening of the recess 23.

The dimension L3 of the recess 23 is preferably in a range of 5 to 25 mm and more preferably in a range of 10 to 20 mm. The dimension L4 of the recess 23 is preferably in a range of 5 to 25 mm and more preferably in a range of 10 to 20 mm.

The depth of the recess 23 is preferably in a range of 1 to 20 mm and more preferably in a range of 4 to 12 mm.

An interval L8 (See Fig. 3) between the adjacent recesses 23 measured in the circumferential direction of the rotary drum 2 is preferably in a range of 0.5 to 10 mm and more preferably in a range of 1 to 5 mm.

An interval L9 (See Fig. 3) between the adjacent recesses 23 measured in the width direction is preferably in a range of 0.5 to 10 mm and more preferably in a range of 1 to 5 mm.

The dimension L6 of the suction pore 53 (same as the dimension of the aforementioned suction region) is preferably in a range of 5 to 25mm and more preferably in a range of 10 to 20mm. The dimension L7 of the suction pore 53 is preferably 5 to 25mm and more preferably in a range of 10 to 20mm.

An interval L10 between the adjacent suction pores 53 (See Fig. 4) measured in the circumferential direction (direction 5X, i.e., the conveying direction of the conveyor system) is preferably in a range of 0.5 to 10mm and more preferably in a range of 1 to 5mm.

An interval L11 (See Fig. 4) between the adjacent suction pores 53 measured in the width direction of the transfer roll 5 (direction 5Y, i.e., the direction orthogonal to the conveying direction of the conveyor system) is preferably in a range of 0.5 to 10mm and more preferably in a range of 1 to 5mm.

From the view point of smooth transfer onto the conveyor system and smooth conveyance by the conveyor system, the aforementioned dimensions preferably are in a relationships of L3≥L6, L4≥7, L8≤L10, L9≤L11 and more preferably are in a relationship of L3>L6, L4>L7, L8<L10 and L9<L11 and it is even more preferable that the aforementioned dimensions meet such relationship.

In the manufacturing method and the manufacturing apparatus according to this embodiment, the paired windbreak plates 9, 9 each set up close to the transfer roll 5 prevent or restrict an air or wind from flowing from bilateral outsides toward region 5M having the suction pores 53, and thereby further reliably prevent the problems such that the accumulations lying in the vicinity of both ends of the opposite side edges of the roll 5 in the width direction thereof might slump against accumulations lying in the midsection of the roll and/or the positional relationship of the accumulations might be disturbed. While not specified, the material of the windbreak plates 9 may be metallic material or synthetic resin and have a thickness preferably in a range of 0.5 to 10mm from the viewpoint of wind-resistant rigidity.

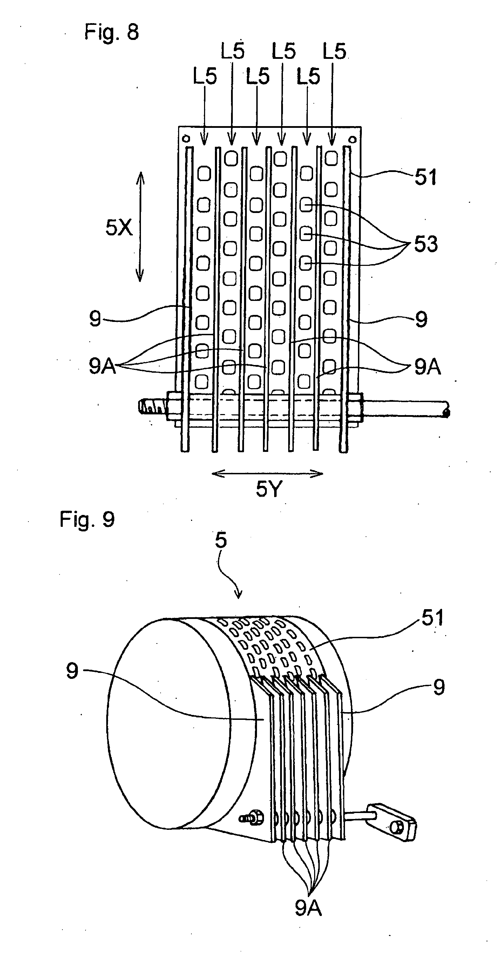

Figs. 8 and 9 illustrate a further preferable embodiment (second embodiment) of this invention.

According to the second embodiment, a plurality of the columns L5 of suction pores including a plurality of the suction pores 53 serially arranged in the circumferential direction (direction 5X) are formed in the width direction (direction 5Y) of the transfer roll in a similar way to the aforementioned first embodiment. The second embodiment is distinguished from the first embodiment in that inter-column partition plate 9A is provided between each pair of the adjacent columns L5, L5 of suction pores. The accumulations 32 retained on the transfer roll 5 and conveyed move on pathways defined between the adjacent inter-column partition plates 9A or on the pathway defined between the inter-column partition plate 9A and the associated windbreak plate 9. The inter-column partition plates 9A function as slump preventing guides and further reliably prevent the accumulations 32 from slumping against the adjacent accumulations. The features of the second embodiment which are not specifically described here are same as those of the aforementioned first embodiment. While the inter-column partition plates 9A have similar shapes as the aforementioned windbreak plates 9, it is not essential for the inter-column partition plates 9A to have same shape as the shape of the windbreak plates 9.

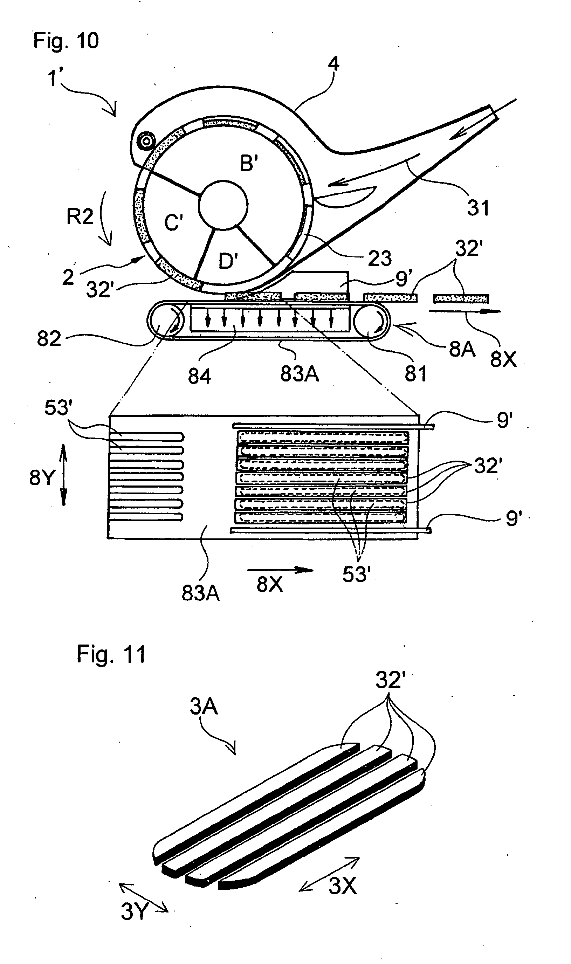

Fig. 10 is a diagram illustrating a third embodiment of this invention.

An absorbent member manufacturing apparatus 1' illustrated in Fig. 10 includes the rotary drum 2 having the recesses 23 on the outer peripheral surface which each have a plurality of fine pores on the bottom thereof and adapted to be rotationally driven in the direction of the arrow R2, the duct 4 serving to feed fibrous materials 31 for the absorbent member onto the outer peripheral surface of the rotary drum 2, the vacuum conveyor 8A set up below the rotary drum 2 and the windbreak plates 9' set up close to the vacuum conveyor 8A. The apparatus 1' is configured to supply the fibrous materials 31 onto the outer peripheral surface of the rotary drum 2 and accumulate the fibrous materials 31 in the recesses, to release accumulations from the respective recesses 23 under the suction force provided through the suction pores 53' formed through the vacuum conveyor 8A and to transfer the accumulations onto the vacuum conveyor 8A so that the accumulations 32 may be then conveyed by the vacuum conveyor 8A as the accumulation 32 are retained thereon.

The rotary drum 2 in the manufacturing apparatus 1' illustrated in Fig. 10 is also formed on the outer peripheral surface with a plurality of recesses into which the fibrous material 31 for the absorbent member are accumulated under the suction force wherein these recesses 23 are spaced from each other in the circumferential direction as well as in the width direction. In this regard, the recesses 23 spaced from each other in the circumferential direction correspond to a plurality of absorbent articles (e.g., disposable diapers or sanitary napkins).

More specifically, the rotary drum 2 in the manufacturing apparatus 1' illustrated in Fig. 10 also is formed with a plurality of the recess groups 23 spaced from each other in the circumferential direction of the rotary drum 2 each consisting of a plurality of the recesses 23 adapted to form the accumulations 32' to be included in the individual absorbent articles, respectively. The recesses 23 included in the respective recess groups are spaced from each other only in the width direction of the rotary drum 2 but are continuous with each other in the circumferential direction of the rotary drum 2. As will be apparent from the absorbent member (or absorbent core) 3A obtained with use of the apparatus for the individual absorbent article as illustrated in Fig. 11, the accumulations 32' constituting this absorbent member 3A have a shape relatively long in the circumferential direction of the rotary drum 2 and in the longitudinal direction (direction 3X) corresponding to the conveying direction 8X of the vacuum conveyor 8 and a plurality of the accumulations are arranged only in the width direction (direction 3Y) being orthogonal to the longitudinal direction.

The vacuum conveyor 8A in the manufacturing apparatus 1' of Fig. 10 includes an endless patterned suction conveyor belt 83A put on a driving roll 81 and a driven roll 82 and this patterned suction conveyor belt 83A is formed with suction pores 53' associated with the respective recesses 23 of the rotary drum 2. In the course of running over the vacuum box 84, the patterned suction conveyor belt 83A provides the suction force from the suction pores 53'.

As illustrated in Fig. 10, each of the suction pores 53' of the patterned suction conveyor belt 83A has a planar shape smaller than each of the recesses 23 of the rotary drum 2 and each of the accumulations 32' having been released from the recesses 23. On the outer peripheral surface (particularly above the suction pores 53') of the patterned suction conveyor 83A and within the suction pores 53', it is desirable to provide appropriate members (e.g., meshes) adapted to prevent the fibrous material or the like from being sucked therethrough.

When it is desired to manufacture the absorbent member 3A consisting of a plurality of the accumulations 32' arranged only in the direction orthogonal to the conveying direction as illustrated in Fig. 11, it is possible to arrange serially a plurality of relatively short suction pores 53' formed on patterned suction conveyor belt 83A in the direction corresponding to the longitudinal direction of the one relatively long recess 23 instead of providing the single long suction pore 53' in association with the one relatively long recess 23. In this case also, each suction pore column consisting of a plurality of the suction pores is preferably formed so that the suction pore column might not run off the edges of the one relatively long recess in the conveying direction of the accumulations as well as in the direction orthogonal to the conveying direction.

The windbreak plates 9' are set up in pairs on opposite sides in the width direction (direction Y) of the patterned suction conveyor belt 83A across the region thereof in which the suction pores 53' are formed. From the viewpoint of smooth transfer of the accumulations from the rotary drum to the vacuum conveyor 8A, the suction force provided from the bottoms of the respective recesses 23 is preferably controlled to be lower during passage of the recesses 23 of the rotary drum 2 above the space C' than during passage of the recesses 23 above the space B' and to be stopped during passage of the recesses 23 above the space D'.

According to the manufacturing apparatus 1' and the manufacturing method with use of this apparatus 1' for the absorbent member 3A, the accumulations 32' within a plurality of the recesses 23 of the rotary drum 2 are individually subjected to the suction force provided from the suction pores 53' associated with the respective recesses 23 to transfer these accumulations 32' onto the vacuum conveyor 8A and these released accumulations 32' are conveyed while being individually sucked. In this way, the operating effect equivalent to that of the aforementioned manufacturing apparatus 1 and the manufacturing method with use thereof can be achieved.

While the method and the apparatus according to this invention for manufacturing of the absorbent member has been described hereinbefore on the basis of some specific embodiments, this invention is not limited to these embodiments and these embodiments may be appropriately modified and varied.

For example, while Figs. 3 and 4 illustrate the embodiment in which a plurality of the recess groups 23G and a plurality of the suction pore groups 53G are arranged intermittently in the circumferential direction on the outer peripheral surfaces of the rotary drum 2 and the transfer roll 5, respectively, it is possible to form the outer peripheral surfaces of the rotary drum 2 and the transfer roll 5 with the recess columns L2 and the suction pore columns L5 continuously arranged over all circumferences.

When a plurality of the recess groups 23G and a plurality of the suction pore groups 53G each of these groups 23G, 53G corresponding to the absorbent member for each of the individual absorbent articles are arranged in the circumferential direction of the rotary drum 2 and the transfer roll 5, the number of the recesses 23 forming each of the recess columns L2 in the recess groups 23G (8 in the embodiment illustrated in Fig. 3) or the number of the suction pores 53 forming each of the suction pore columns L5 in the suction pore groups 53G (9 in the embodiment illustrated in Fig. 4) may be in a range of 1 to 7 or in a range of 10 to 20.

The recess groups and the suction pore groups corresponding to each of the individual absorbent articles may include the recess columns and the suction pore columns may include the recess columns and the suction pore columns extending at an angle to the conveying directions of the conveying means such as the rotary drum 2, the transfer roll 5 and the vacuum conveyor 8A.

While both the number of the recess columns L2 arranged in the direction 2Y and the number of the suction pore columns L5 arranged in the direction 5Y is seven (7) in the embodiment illustrated in Figs. 3 and 4, the number of the recess columns L2 and the suction pore columns L5 may be in a range of 2 to 6 or in a range of 8 to 30. In this regard, the number of both the recess columns and the suction pore columns are preferably 3 or more and more preferably 4 or more.

When a plurality of the recess columns L2 and a plurality of the suction pore columns L5 are arranged, the circumferential positions (in the direction 2X or the direction 5X) of the adjacent recesses 23 or suction pores 53 in each pair of the adjacent recess columns L2 or the adjacent suction pore columns L5 may be in alignment or out of alignment (direction 2X or direction 5X). Preferably, the circumferential positions of the recesses 23 or the suction pores 53 in the all recess or suction pore columns are in alignment.

Instead of transferring the accumulations 32 within the recesses onto the mesh belt and the like being supplied onto the transfer roll 5, it is possible to transfer the accumulations directly onto the outer peripheral surface of the transfer roll 5 adapted to be not supplied with the mesh belt 7. In this case, the outer periphery of the transfer roll 5 is preferably provided over or within the suction pores 53 with an appropriate member (e.g., mesh) in order to prevent the fibrous material or the like from being sucked in the suction pores. Instead of supplying the mesh belt 7 onto the transfer roll 5, it is possible to supply the core wrapping sheet such as the second core wrapping sheet 34 onto the transfer roll 5 and to transfer the accumulations 32 onto this core wrapping sheet.

It is also possible to form the mesh belt with air-permeable zones and non-air-permeable zones and thereby to replace the suction pores by these air-permeable zones.

The absorbent member manufactured according to this invention may have the form of the accumulations 32 of which the upper and lower surfaces are wrapped with different core wrapping sheets 33, 34 as in the case of the aforementioned embodiment. Alternatively, both surfaces of the accumulations 32 may be wrapped with a single core wrapping sheet, for example, one of the surfaces of these accumulations 32 may covered with a central portion of the single core wrapping sheet and the other surface may be wrapped with the remaining portions of this single core wrapping sheet folded back onto the other surface of the accumulations. It is also possible to fix a plurality of the accumulations 32 to one surface side of a single core wrapping sheet, for example, by means of adhesive and the other surface side may be free of wrapping. Furthermore, the absorbent member may have the form of a multilayered absorbent member including a relatively large absorbent layer and a plurality of the accumulations 32 stacked thereon.

After the accumulations 32 have been conveyed by the transfer roll 5, it is also possible to transfer the accumulations onto a belt conveyor having no suction mechanism or onto the other conveying means. The shape of the suction pore 53 is not limited to the shape which is same as or similar to the shape of the recess 23. The cross-sectional shape of the suction pore 53 or the shape of the periphery of opening thereof may be selected from various shapes such as circular, oval and rhombic shapes.

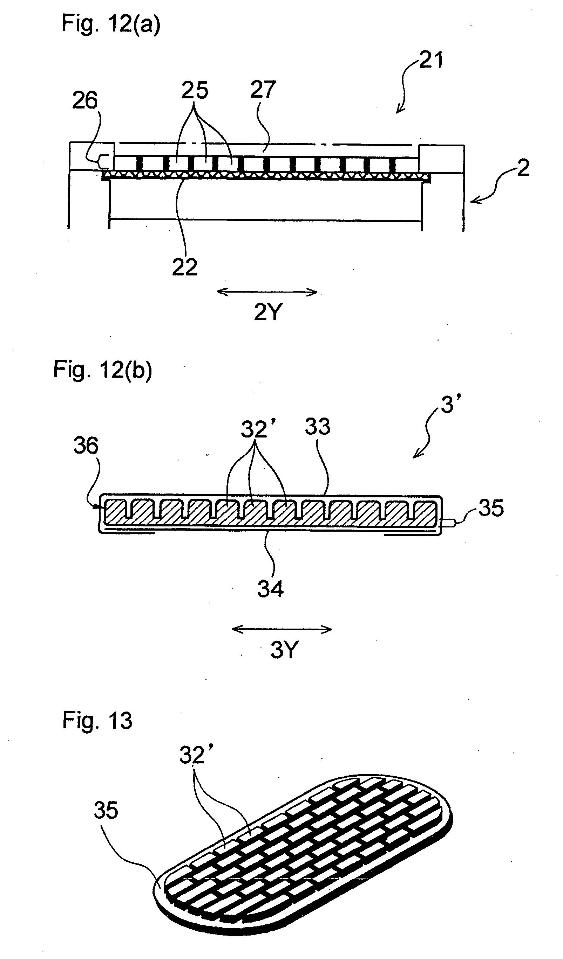

The recesses of the rotary drum in this invention may have an alternative configuration as illustrated in Fig. 12 (a) including a lower layer 26 defined by a plurality of small recesses 25 divided in the width direction (direction 2Y) or in the width direction and the circumferential direction of the rotary drum 2 and an upper layer 27 spanning these small recesses 25. Fig. 12 (b) exemplarily illustrates the absorbent member manufactured with use of the rotary drum illustrated in Fig. 12 (a). The absorbent member 3' illustrated in Fig. 12 (b) has the absorbent core 36 consisting of convex portions 32' formed from the accumulations in the small recesses 25 and a continuous layer 35 formed from accumulations accumulated in the upper layer 27. Fig. 13 exemplarily illustrates the absorbent member obtained with use of the rotary drum 2 having the recesses including the lower layer 26 consisting of a plurality of the small recesses 25 divided in the width direction as well as in the circumferential direction.

The transfer roll and the vacuum conveyor may be formed with the suction pores associated with the respective small recesses 25 to prevent the convex portions 32' formed from the accumulations in the small recesses 25 from slumping against the adjacent accumulations and/or collapsing.

This invention will be described hereunder on the basis of a fourth embodiment thereof.

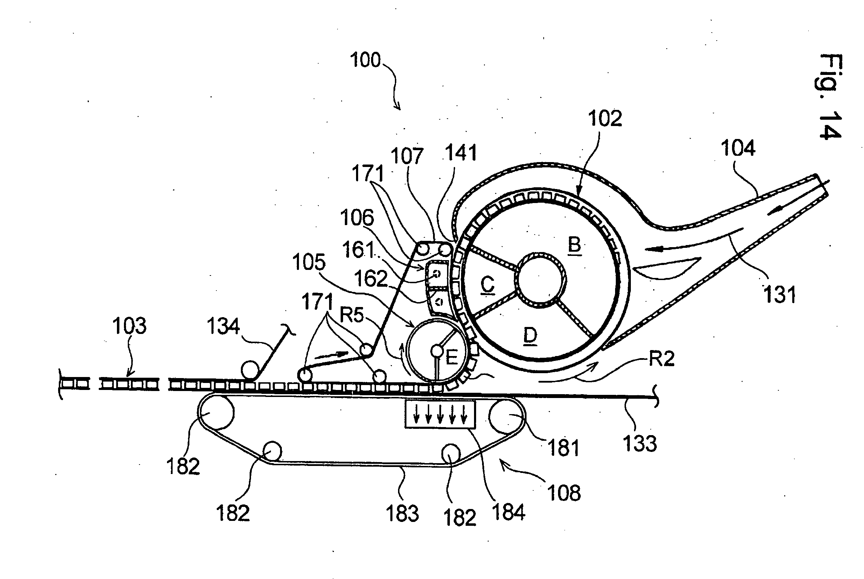

An apparatus 100 according to the fourth embodiment of this invention used to manufacture the absorbent members includes, as illustrated in Fig. 14, a rotary drum 102 adapted to be rotationally driven in a direction of an arrow R2, a duct 104 serving to feed fibrous material 131 as basic ingredient of the absorbent member 103 onto an outer peripheral surface of the rotary drum 102, a transfer roll 105 set up obliquely below the rotary drum 102 and adapted to be rotationally driven in a direction of an arrow R5, a vacuum box 106 set up between the duct 104 and the transfer roll 105 as viewed in the peripheral direction of the rotary drum 102, sheet-like air-permeable mesh belt 107 set up so as to pass between the vacuum box 106 and the rotary drum 102 and between the transfer roll 105 and the rotary drum 102, and vacuum conveyor 108 set up below the transfer roll 105.

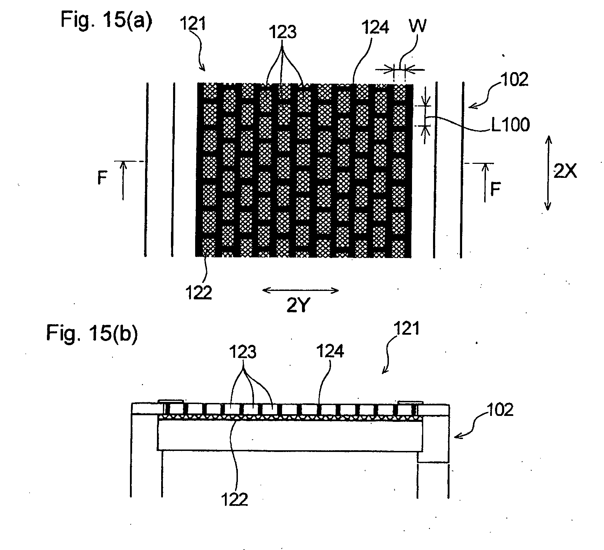

As illustrated in Fig. 14, the rotary drum 102 has a cylindrical shape and is driven by a power engine such as electric motor to be rotated around a horizontal axis. As illustrated in Fig. 15 (a) and Fig. 15 (b), the rotary drum 102 is formed on its outer peripheral surface 121 with a plurality of recesses 123 having a bottom formed of a mesh plate 122.

As illustrated in Fig. 15, a plurality of the recesses 123 of the rotary drum 102 are spaced from each other in the circumferential direction (direction 2X) as well as in the width direction (direction 2Y) of the rotary drum 102.

More specifically, a plurality of the recess columns each including a plurality of the recesses 123 serially arranged in the circumferential direction (direction 2X) are arranged in the width direction (direction 2Y) of the rotary drum 102.

The respective recesses 123 are divided off by a partition member 124. The partition member 124 is made of a flexible plate formed with a plurality of through-holes each having the same shape as that of the recess 123.

A plurality of the recesses 123 are defined by bending this flexible plate in a circular arc-shape or a cylindrical shape so as to follow the outer peripheral surface of the rotary drum 102 and fixing this plate to the outer peripheral surface by means of fixing means of well known art such as bolts or adhesive in a state of putting the flexible plate on fiber-stacked mesh 122. The inner peripheral surface of the recess 123 is defined by the inner peripheral surface of the through-hole of the flexible plate. In this regard, it is possible to prepare the flexible plate previously treated so as to have the circular arc-shape.

The fiber-stacked mesh 122 has a plurality of fine pores adapted to block the fibrous material for the absorbent member carried on the air flow and to allow the air flow only to pass through the mesh.

An irrotational portion defined inside the rotary drum 102 (on the side of the axis of rotation) is formed with a space B adapted to be depressurized. An air exhaust system of well known art such as an intake fun (not shown) is connected to this space B so that the space B may be maintained at a negative pressure by actuating such air exhaust system.

Also inside the rotary drum 102 (on the side of the axis of rotation), spaces C and D are defined and respectively have pipes connected to these spaces to intake the ambient air.

As illustrated in Fig. 14, the segment of the duct 104 on the side of one end thereof covers the segment of the outer peripheral surface of the rotary drum 102 defined above the space B and the segment of the duct 104 on the side of the other end thereof not illustrated in Fig. 14 includes therein a fibrous material delivery device. The fibrous material delivery device includes, for example, a grinder mill adapted to crush sheet-like wood pulp into fluff pulp (fibrous material) to be delivered into the duct. It is possible to set up a liquid-absorbent polymer delivery device along the duct 104.

During passage of the individual recesses 123 of the rotary drum 102 above the space B maintained at a negative pressure, a suction force is provided through the fine pores formed in the bottoms of the recesses 123. Such suction force provided through the fine pores in he bottoms of the recesses 123 generates air flow serving to feed the material for the absorbent member delivered from the fibrous material delivery device and the water-absorbent polymer delivery device having been delivered into the duct 104 to the outer peripheral surface of the rotary drum 102. The materials fed by the air flow in this manner are accumulated in the recesses 123.

The transfer roll 105 has an air-permeable cylindrical outer periphery and is driven by a power engine such as an electric motor so that the outer periphery is rotated around a horizontal axis. An irrotational portion defined inside the transfer roll 105 (on the side of its axis of rotation) is formed with a space E adapted to be depressurized. An air exhaust system of well known art such as an air intake fun (not shown) is connected to this space E so that the space E may be maintained at a negative pressure by actuating such air exhaust system.

The vacuum box 106 is set up between a downstream end 141 of the duct 104 and the transfer roll 105 as viewed in the rotational direction of the rotary drum 102.

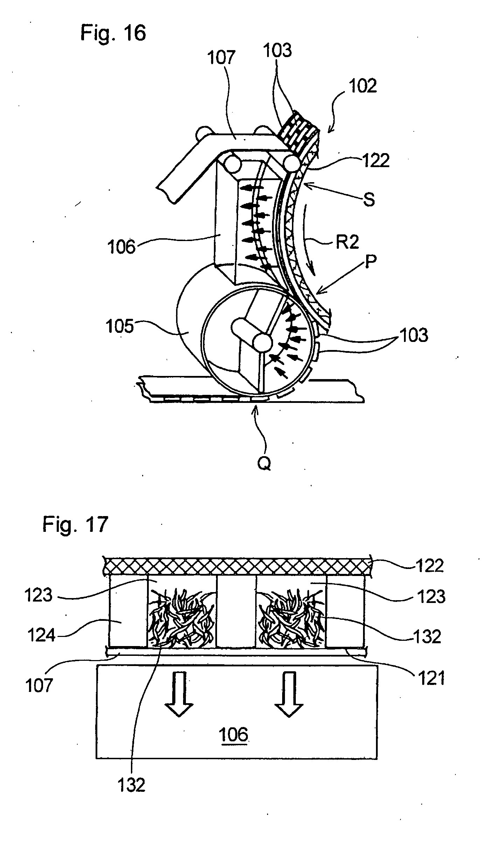

As illustrated in Fig. 16, the vacuum box 106 has a box-like configuration defined by upper and lower surfaces, opposite side surfaces and a rear surface wherein a region opposed to the rear surface is provided with an opening which opens toward the rotary drum 102.

The vacuum box 106 is connected to an air exhaust system (not shown) of well known art such as an air intake fan via an exhaust pipe or the like (not shown) so that the interior of the vacuum box 106 may be maintained at a negative pressure by actuation of the air exhaust system.

The mesh belt 107 is a belt-like air-permeable member, i.e., a mesh belt provided in the form of an endless belt and guided a plurality of free rolls 171 and the transfer roll 105 to run continuously along a predetermined pathway. The mesh belt 107 is driven by rotation of the transfer roll 105. The mesh belt 107 is set up so as to be guided onto the outer peripheral surface of the rotary drum 102 in the vicinity of the downstream side end 141 of the duct 104 as illustrated in Fig. 1, then sequentially to pass between the vacuum box 106 and the rotary drum 102 and between the transfer roll 105 and the rotary drum 102 as illustrated in Figs. 14 and 16

The mesh belt 107 is kept in contact with the outer peripheral surface of the rotary drum 102 during passage of the mesh belt 107 in front of the opening of the vacuum box 106 and, in the vicinity of a region in which the transfer roll 105 and the rotary drum 102 come closest to each other, the mesh belt 107 is transferred from the outer peripheral surface of the rotary drum 102 onto the transfer roll 105.

The vacuum conveyor 108 includes an endless air-permeable belt 183 running along a driving roll 181 and driven rolls 182, 182 and a vacuum box 184 set up at a location opposed to the transfer roll 105 across the air-permeable belt 183.

Now a method of manufacturing the absorbent members 103 in a continuous manner with use of the aforementioned manufacturing apparatus 100 for the absorbent members 103, i.e., a manufacturing method according to this invention for the absorbent member will be described hereunder on the basis of one embodiment thereof.

To manufacture the absorbent members 103 with use of the absorbent member manufacturing apparatus 100, the exhaust systems respectively connected to the space B within the rotary drum 102, the space E within the transfer roll 105 and the space within the vacuum box 106 are actuated to maintain these spaces at negative pressure. The negative pressure maintained within the space B generates an air flow within the duct 104 and this air flow functions to convey the material for the absorbent members toward the outer peripheral surface of the rotary drum 102. The rotary drum 102 and the transfer roll 105 are rotated and the vacuum conveyor 108 also is actuated.

When the fibrous material delivery device is actuated to feed the fibrous material into the duct 104, the fibrous material is scattered and borne on the air flow and fed toward the outer peripheral surface of the rotary drum 102.

In the course of being conveyed immediately under the duct 104, the fibrous material 131 is accumulated in the recesses 123 of the rotary drum 102 under the suction force. In this embodiment, a material feeding zone is a zone which is located in the rotary drum 102 and is covered with the duct 104. Thereupon, the accumulations 132 accumulated in the recesses 123 are shaped so as to follow the shape of the inner peripheral surface of the respective recesses 123.

When these recesses 123 come to a position opposed to the vacuum box 106 as the rotary drum 102 rotates, the accumulations 132 in the respective recesses 123 stick to the mesh belt 107 under the suction force provided from the vacuum box 106. The accumulations 132 within the respective recesses 123 are conveyed in such state to a position immediately near to the position P at which the transfer roll 105 and the rotary drum 102 come closest to each other. In this regard, while it is not essential to maintain a clearance between the accumulation 132 and the bottom of the recess 123 as illustrated in Fig. 17 when accumulation 132 in the recess 123 is sucked to the mesh belt 107, it is preferred to maintain such clearance from the viewpoint of smooth transfer of the accumulations 132 to the other conveying system.

In the vicinity of the position P at which the transfer roll 105 and the rotary drum 102 come closest to each other (See Fig. 16), the accumulations 132 within the respective recesses 123 are released from the recesses 123 in a state of being stick to the mesh belt 107 under the suction force provided from the side of the transfer roll 105 and transferred onto the transfer roll 105.

The accumulations 132 transferred together with the mesh belt 107 onto the transfer roll 105 are conveyed in a state of sticking to the mesh belt 107 to a delivery point Q at which the accumulations 132 are transferred onto the vacuum conveyor 108 under the suction force provided from the vacuum box 184.

In the apparatus 100 according to this embodiment, a first core wrapping sheet 133 such as tissue paper or liquid-permeable nonwoven fabric is introduced onto the vacuum conveyor 108 before the accumulations 132 are transferred onto the vacuum conveyor 108 and then the accumulations 132 are transferred onto this first core wrapping sheet 133 as illustrated in Fig. 14.

Then, a second core wrapping sheet 134 is introduced onto the side of the upper surfaces of the accumulations 132 and then these accumulations 132 wrapped with the first and second wrapping sheets 133, 134 are cut at predetermined intervals corresponding to a dimension of the individual absorbent member to obtain the absorbent member 103.

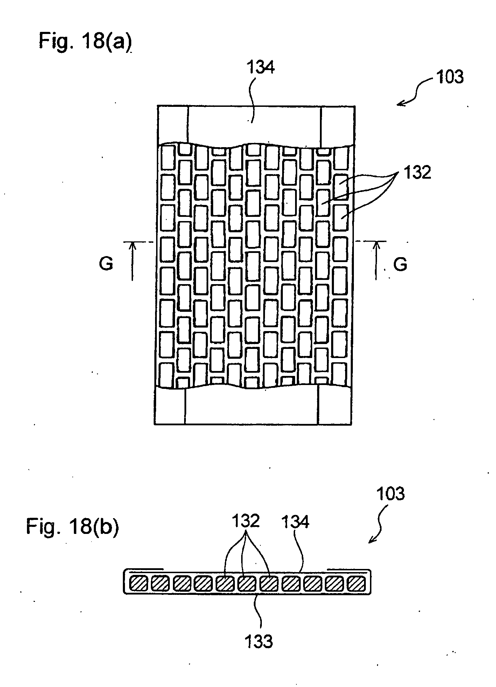

While the first and second wrapping sheets 133, 134 may be bonded to each other without folding back these wrapping sheets 133, 134, opposite lateral portions of one of the sheets may be folded upward or downward to the side of the other wrapping sheet and then fixed together by means of adhesive or the other technique. The absorbent member 103 illustrated in Fig. 18 (a) and Fig. 18 (b) is the absorbent member obtained by folding upward or downward the opposite lateral portions of the first wrapping sheet 133 to the side of the second wrapping sheet 134.

In the case of the manufacturing apparatus and the manufacturing method according to this embodiment, the accumulations 132 are conveyed while these accumulations 132 are subjected to the suction force provided from the vacuum box opposite to the rotary drum 102 before the accumulations 132 are released from the recesses 123 of the rotary drum 102. Consequently, there is almost no anxiety that the accumulations 132 might get out shape or positional relationship of the accumulations might be disturbed in the course of transferring the accumulations 132 onto the transfer roll 105 (the other conveying means).

Particularly in this embodiment, the absorbent member 103 to be manufactured includes a plurality of block-like accumulations (molded product of material) 132 arranged so as to be spaced each other in the longitudinal direction as well as in the width direction. The method of prior art for manufacturing such absorbent member has often been accompanied with the problem such that the positional relationship among the accumulations might be disturbed in the course of transferring the accumulations 132 from the recesses 123 onto the other conveying means. In contrast, according to this embodiment of this invention, the accumulations 132 are conveyed while these accumulations 132 are subjected to the suction force provided from the vacuum box opposite to the rotary drum 102 before the accumulations 132 are released from the recesses 123 of the rotary drum 102. In this way, it is possible to arrange the accumulations on the other conveying means as if the pattern of the recesses 123 has been exactly transcribed onto the other conveying means and thereby to obtain the absorbent member 103 consisting of a plurality of the accumulations 132 orderly arranged at desired positions with a high degree of accuracy. In the absorbent member 103 illustrated in Fig. 18, the accumulations (molded product of the material) 132 are arranged in a zigzag pattern. In this regard, it is possible to form the absorbent member on its longitudinally opposite ends with regions each having a predetermined width in which the accumulations 132 are not present. It is possible to incorporate the absorbent member 103 illustrated in Fig. 18 into the absorbent article so that the longitudinal direction of the absorbent member 103 corresponding to the direction MD in the manufacturing process may correspond to the front-back direction of the absorbent article such as disposable diaper, sanitary napkin or incontinent pad or the direction of the absorbent member 103 corresponding to the direction CD in the manufacturing process may correspond to the front-back direction of the absorbent article. The front-back direction of the absorbent article corresponds to the front-back direction of the article wearer when this absorbent article is put on the wearer's body.

While a length L100 of the recess 123 in the circumferential direction (direction 2X) of the rotary drum 102 (as measured along the outer peripheral surface of the rotary drum) and a length W of the recess 123 in the width direction (direction 2Y) of the rotary drum 102 are not specified, for example, assuming that the absorbent member consisting of a plurality of the accumulations (molded product of material) 132 arranged so as to be spaced from each other is manufactured, the length L100 may be, for example, in a range of 1.5 to 15cm and preferably in a range of 5 to 10cm. The length W may be, for example, in a range of 1 to 10cm and preferably in a range of 2 to 5cm. The length dimensions of the accumulation (molded product of the material) 132 in the finished absorbent member 103 in the direction 2Y and the direction 2X are also preferably in the similar ranges.

A width of the region to be subjected to the suction force by the vacuum box 106 (the length in the direction corresponding to the width direction of the rotary drum) is same as or larger than the width of the rotary drum provided with the recesses (a length of the rotary drum in its width direction).

From the viewpoint of preventing the accumulations from getting out shapes and preventing the positional relationship among the accumulations from being disturbed, a distance over which the respective accumulations 132 are conveyed under the suction force provided from the side opposite to the rotary drum 102 (referred to hereunder simply as a conveyed distance under suction) is preferably 1.5 times or more of a length of the respective recesses 123 in the circumferential direction of the rotary drum (measured along the outer peripheral surface of the rotary drum), more preferably in a range of 1.5 to 10 times of this length and even more preferably in a range of 2 to 5 times of this length. The conveyed distance under suction corresponds to a distance from a point S (See Fig. 16) at which the respective recesses 123 containing the accumulations 132 therein are subjected for the first time to the suction force from the side opposite to the rotary drum 102 to the position P at which the transfer roll 105 and the rotary drum 102 come closest to each other.

When the accumulations 132 are released from the recesses 123 directly onto the vacuum conveyor 108 without using the transfer roll 105, the conveyed distance corresponds to the distance from the point S to the position at which the transfer roll 105 and the rotary drum 102 come closest to each other.

It is possible to release the accumulations 132 from the recesses 123 directly onto the vacuum conveyor 108 instead of releasing the accumulations 132 from the recesses 123 onto the transfer roll 105. However, from the viewpoint of preventing the accumulations 132 in the recesses 123 from getting out shapes and preventing the positional relationship of the accumulations 132 from being disturbed, it is preferred to release the accumulations 132 onto the transfer roll 105 and then to transfer the accumulations 132 onto the vacuum conveyor 108 as in this embodiment.

It is also possible to supply a core wrapping sheet such as the second core wrapping sheet 134 onto the recesses 123 instead of supplying the endless mesh 107 onto the recesses 123, then to convey the accumulations 132 maintained sticking to the core wrapping sheet under the suction force and to transfer the accumulations 132 onto the other conveying means such as the transfer roll 105 or the vacuum conveyor 108. However, from the viewpoint of preventing the accumulations 132 in the recesses 123 from getting out shapes and preventing the positional relationship of the accumulations 132 from being disturbed due to a variation in conveying tension, it is preferred to use the endless mesh belt 107 and thereby to assure stabilized suction force as in this embodiment.

The vacuum box 106 is preferably provided therein with rectifying plates so that the air flow sucked from the surface of the box 106 into the interior of the box 106 may be rectified. In this way, the accumulations 132 within the recesses 123 can be evenly sucked toward the endless mesh belt 107 and thereby deformation and position disturbance of the transferred accumulations 132 can be prevented.

As the rectifying plates, for example, a plurality of plates may be mounted within the vacuum box so as to be opposed to the openings of the vacuum box and to be substantially perpendicular to the sheet-like air-permeable member or a honeycomb panels may be mounted within the vacuum box so as to be opposed to the openings of the vacuum box and to define cylindrical air flow channels substantially perpendicular to the sheet-like air-permeable member. It is also possible to arrange these plates so as to extend vertically and in parallel to each other or to combine these plates so as to form a plurality of air flow channels each having triangular, rectangular or rhombic cross-sectional shape.

The interior of the vacuum box 106 is preferably divided into a plurality of small spaces arranged in the circumferential direction of the rotary drum 102. A plurality of the small spaces arranged in the circumferential direction of the rotary drum allows the suction force in the course of the conveying under suction to be regulated and thereby to stabilize the conveyance.

The number of the small spaced arranged in the circumferential direction of the rotary drum 102 is at least two and the suction force from these small spaces is preferably different depending on the respective small spaces.

The number of the small spaces arranged in the circumferential direction of the rotary drum 102 is preferably 2 or more and more preferably in a range of 2 to 5. In this regard, Fig. 14 illustrates the vacuum box 106 of which the interior is divided by a partition wall 162 into two small spaces.

From the viewpoint of improvement in transferability of the accumulations 132, a moving velocity of the mesh belt 107 when the mesh belt 107 (sheet-like air-permeable member) passes through the position P at which the transfer roll 105 and the rotary drum 102 come closest to each other is preferably 102 or less and more preferably 95 or more and less than 100 relative to the moving velocity of the rotary drum assumed as 100.

A clearance between the transfer roll 105 and the rotary drum 102 (more strictly, a distance between the mesh belt 107 in contact with the outer peripheral surface of the transfer roll 105 and the outer peripheral surface of the rotary drum 102) is preferably in a range of 3.0 to 7.0mm and more preferably in a range of 5.0 to 6.5mm.

While the fourth embodiment of the method and the apparatus according to this invention for manufacturing the absorbent member has been described hereinbefore, this invention is not limited to the aforementioned embodiment and may be appropriately varied and modified.

For example, while the rotary drum 102 illustrated in Fig. 15 (a) is formed with a plurality of the recess columns (referred to hereunder also as the circumferential recess columns) each consisting of a plurality of the recesses 123 serially arranged in the circumferential direction (direction 2X) of the rotary drum in the axis direction (direction 2Y) of the rotary drum, it is also possible to form a single circumferential recess column in the axis direction (direction 2Y) of the rotary drum. It is also possible to form the one and same rotary drum 102 with both the circumferential recess columns and the groove-like recesses continuously formed in the circumferential direction (direction 2X) of the rotary drum.

When a plurality of the circumferential recess columns is formed, the circumferential positions of the recesses in the all the circumferential recess columns are in alignment or those in the adjacent the circumferential recess columns are out of alignment as viewed in the axial direction of the rotary drum. The circumferential recess column may include a plurality of the recesses different in lengths.

When the outer peripheral surface of the rotary drum 102 is formed with a plurality of types of the recesses having different lengths, the aforementioned conveying distance under suction is preferably regulated to the aforementioned rate on the basis of the length of the shortest recess.

It is possible to form the outer peripheral surface of the rotary drum 102 with regions including the recesses and the regions having no recess in alternate manner and to form the circumferential recess columns having the various patterns described above only in the regions including the recesses. For example, the outer peripheral surface of the rotary drum 102 may be formed with a plurality of the recess groups each used to form the number of the accumulations for each of the absorbent articles so as to be spaced from each other in the circumferential direction, and the recesses in the recess groups may have the various patterns described above.

Also, the recesses 123 may be formed on the rotary drum 102 so as to have a given pitch over all circumferences of the rotary drum 102. A plurality of regions which have the recesses 123 arranged at given pitch may be formed on the rotary drum 102 in the circumferential direction thereof.

Also, the accumulations 132 may have both surfaces wrapped with the different core wrapping sheets 133, 134 as the absorbent member 103 manufactured according to the aforementioned embodiment is the case, or the entire surfaces of the accumulations 132 may be wrapped with a single core wrapping sheet, for example, one surface of the accumulations 132 is wrapped with a central segment of the single core wrapping sheet and the opposite surface of the accumulations 132 are wrapped with both lateral segments of the aforementioned single core wrapping sheet folded onto the opposite surface of the accumulations 132. It is also possible to fix a plurality of the accumulations 132 to one surface of a single sheet by means of adhesive or the like and to leave the other surface of the accumulations not wrapped with any core wrapping sheet.

The feature of which description was eliminated in one embodiment and the feature included in one embodiment alone may be appropriately incorporated into the other embodiments and the features included in the respective embodiments may be mutually replaced among the respective embodiments.

For example, arrangement of the suction pores 53' in the recesses 23 and the belt conveyor 8A in the apparatus adapted to release the accumulations from the recesses 23 of the rotary drum onto the belt conveyor 8A as in the apparatus 1' illustrated in Fig. 10 may be changed to the same arrangement as the arrangement of the recesses 23 and the suction pores 53 in the apparatus 1 illustrated in Figs. 1 and 2. By contraries, the arrangement of the recesses 23 and the suction pores 53 in the apparatus 1 illustrated in Figs. 1 and 2 may be changed to the same arrangement as the arrangement of the recesses 23 and the suction pores 53' in the apparatus 1' of Fig. 10.

Fibrous material as the material for the absorbent member may be selected from various kinds of fibrous material conventionally used for the absorbent member in the absorbent articles such as disposable diaper without any limitation. For example, short fiber obtained from cellulose fiber such as pulp fiber in the form of fluff pulp, rayon fiber and cotton fiber and short fiber of synthetic fiber such as polyethylene fiber may be used. These fibers may be used independently or in combination two or more kinds of these fibers. As the material, in addition to the fibrous material, water-absorbent polymer particles may be introduced into the duct and, if desired, it is also possible to feed deodorant or antibacterial agent together with the fibrous material.

The absorbent member manufactured according to this invention is preferably used as the absorbent member for the absorbent articles. The absorbent articles are primarily used to absorb and to retain bodily fluids excreted from the human body such as urine or menstrual blood. While the absorbent articles include, for example, disposable diaper, sanitary napkin, incontinent pad and panty liner, the intended purposes are not limited to them and more widely include all articles adapted to absorb all kinds of bodily fluids excreted from the human body.

The absorbent articles typically include a topsheet, a backsheet and a liquid-retaining absorbent member sandwiched between these two sheets. The topsheet may serve also as the core wrapping sheet adapted to cover the surface of the absorbent member. The backsheet may have a vapor-permeability also. The absorbent articles may further include various members for various purposes. Such additional members are well known to those skilled in the art. For example, when the absorbent articles are used in the form of disposable diaper or sanitary napkin, it is possible to set up one or more pairs of three-dimensional guards outside the raised opposite sides of the absorbent member.

## Claims

1. A method for manufacturing an absorbent member comprising an accumulating step comprising accumulating material for the absorbent member carried by the air flow in recesses (23, 123) formed on an outer peripheral surface of a rotary drum (2, 102) by sucking, and a conveying step comprising transferring accumulations (32, 32', 132) in the recesses (23, 123) onto conveying means and conveying the accumulations while the accumulations (32, 32', 132) being retained on the conveying means, wherein:
in the accumulating step, the material for the absorbent member is accumulated in a plurality of the recesses (23, 123) formed on the rotary drum (2, 102) so as to be arranged in a width direction of the rotary drum (2, 102); and
in the conveying step, the accumulations (32, 32', 132) are released from the recesses (23, 123) onto the conveying means under a suction force provided from the conveying means and the released accumulations (32, 32', 132) are conveyed while being sucked, wherein the suction force is provided from a plurality of suction pores (53, 53') arranged in the conveying means in a direction orthogonal to its conveying direction so as to be associated with the respective recesses (23, 123) and the accumulations (32, 32', 132) having been released are conveyed while being individually sucked.

2. The method for manufacturing the absorbent member according to Claim 1, further including a step of making the accumulations (32, 32', 132) in the recesses (23, 123) stick to a sheet-like air permeable member supplied onto the recesses (23, 123) and conveying the accumulations (32, 32', 132) in this state before the accumulations (32, 32', 132) are transferred to the conveying means.

3. The method for manufacturing the absorbent member according to Claim 1 wherein:
in the accumulating step, the material for the absorbent member is accumulated in a plurality of the recesses (23, 123) formed on the rotary drum (2, 102) so as to be arranged in the circumferential direction and in the width direction, respectively; and
in the conveying step, the accumulations (32, 32', 132) are released from the recesses (23, 123) onto the aforementioned conveying means under the suction force provided from a plurality of the suction pores (53, 53') formed in the conveying means so as to be arranged in a conveying direction and in a direction orthogonal to this conveying direction and to be associated with the individual recesses (23, 123) and the accumulations (32, 32', 132) having been released are conveyed while being individually sucked.

4. The method for manufacturing the absorbent member according to Claim 1 or 3 wherein the conveying means is defined by a transfer roll (5, 105) and a mesh belt (7, 107) provided on an outer peripheral surface of the transfer roll, or a vacuum conveyor (8, 8A,108).

5. An apparatus for manufacturing an absorbent member comprising a rotary drum (2, 102) having recesses (23, 123) on its outer peripheral surface which have a plurality of fine pores on respective bottoms, and being configured to accumulate material for the absorbent member supplied from a material feeding zone in the respective recesses (23,123), to release accumulations (32, 32', 132) from the recesses (23, 123) and to transfer them onto conveying means so that the accumulations (32, 32', 132) may be conveyed while being retained on the conveying means, wherein:
a plurality of the recesses (23, 123) are arranged so as to be spaced from each other in a circumferential direction as well as in a width direction of the rotary drum (2, 102),wherein the conveying means is provided with suction pores (53, 53') arranged in the conveying means in a direction orthogonal to its conveying direction and individually associated with a plurality of the recesses (23, 123) so that these suction pores (53, 53') may individually suck the accumulations (32, 32', 132) in the recesses.

6. The apparatus for manufacturing the absorbent member according to Claim 5, wherein each of the suction pores (53, 53') has a dimension in a direction orthogonal to the conveying direction equal to or smaller than a dimension of each of the recesses (23, 123) in the width direction of the rotary drum (2, 102) and has a dimension in the conveying direction equal to or smaller than a dimension of each of the recesses (23, 123) in the circumferential direction of the rotary drum (2, 102).

7. The apparatus for manufacturing the absorbent member according to Claim 5 or 6, wherein a windbreak plate (9, 9') set up in the vicinity of the conveying means serves to prevent or alleviate the wind which would otherwise flow into a region including the suction pores in a direction orthogonal to the conveying direction.

8. The apparatus for manufacturing the absorbent member according to Claims 5, 6, or 7, wherein a plurality of suction pore columns each including a plurality of the suction pores (53, 53') serially arranged in the conveying direction of the aforementioned conveying means are formed in the direction orthogonal to the conveying direction, and an inter-column partition plate is provided between each pair of the adjacent suction pore columns.

9. The apparatus for manufacturing the absorbent member according to any one of Claims 5 through 8, wherein the conveying means is defined by a transfer roll (5, 105) and a mesh belt provided on an outer peripheral surface of the transfer roll (5, 105), or a vacuum conveyor (8, 8A, 108).

10. The apparatus for manufacturing the absorbent member according to Claim 5, wherein the conveying means is defined by a transfer roll (5, 105) and a mesh belt (7, 107) provided on the outer peripheral surface of the transfer roll (5, 105), and a vacuum box (6, 106) is located between the material feeding zone and the transfer roll (5, 105) so that the vacuum box (6, 106) sucks the accumulations (32, 32', 132) in the recesses (23, 123) from the side opposite to the side of the rotary drum (2, 102).

11. The apparatus for manufacturing the absorbent member according to Claim 10 wherein the mesh belt (7, 107) is adapted to pass between the vacuum box (6, 106) and the transfer roll (5, 105) in a continuous manner.

## Patentansprüche

1. Verfahren zum Herstellen eines absorbierenden Elements, mit einen Akkumulationsschritt, bei dem vom Luftstrom transportiertes Akkumulations-Material für das absorbierende Element in an der äußeren Umfangsoberfläche einer Drehtrommel (2, 102) gebildeten Ausnehmungen (23, 123) durch Ansaugen angereichert wird, und einem Förderschritt, bei dem Akkumulationen (32, 32', 132) in den Ausnehmungen (23, 123) auf eine Fördereinrichtung übertragen werden und die Akkumulationen befördert werden, während die Akkumulationen (32, 32', 132) auf der Fördereinrichtung gehalten werden, wobei:
in dem Akkumulationsschritt das Material für das absorbierende Element in mehreren der an der Drehtrommel (2, 102) gebildeten Ausnehmungen (23, 123) so akkumuliert wird, dass es in einer Breitenrichtung der Drehtrommel (2, 102) angebracht ist; und
in dem Förderschritt die Akkumulationen (32, 32', 132) aus den Ausnehmungen (23, 123) unter einer von der Fördereinrichtung bereitgestellten Saugkraft auf die Fördereinrichtung freigesetzt werden und die freigesetzten Akkumulationen (32, 32', 132) während des Ansaugens befördert werden, wobei die Saugkraft von mehreren Saugporen (53, 53') bereitgestellt wird, die auf der Fördereinrichtung in einer Richtung rechtwinklig zu deren Förderrichtung angebracht sind, so dass sie mit den jeweiligen Ausnehmungen (23, 123) verbunden sind und die freigesetzten Akkumulationen (32, 32', 132) befördert werden, während sie einzeln angesaugt werden.

2. Verfahren zur Herstellung des absorbierenden Elements nach Anspruch 1, ferner mit einem Schritt zum Befestigen der Akkumulationen (32, 32', 132) in den Ausnehmungen (23, 123) an einem schichtähnlichen, auf den Ausnehmungen (23, 123) aufgebrachten luftdurchlässigen Element, und zum Befördern der Akkumulationen (32, 32', 132) in diesem Zustand, bevor die Akkumulationen (32, 32', 132) auf die Fördereinrichtung übertragen werden.

3. Verfahren zur Herstellung des absorbierenden Elements nach Anspruch 1, wobei:
im Akkumulationsschritt das Material für das absorbierende Element in mehreren der an der Drehtrommel (2, 102) gebildeten Ausnehmungen (23, 123) so angereichert wird, dass es in Umfangsrichtung bzw. in Breitenrichtung angeordnet ist; und
in dem Förderschritt die Akkumulationen (32, 32', 132) aus den Ausnehmungen (23, 123) auf die vorgenannte Fördereinrichtung freigesetzt werden unter der Saugkraft, die von mehreren der Saugporen (53, 53') bereitgestellt wird, die so in der Fördereinrichtung ausgebildet sind, dass sie in der Förderrichtung und in einer Richtung rechtwinklig zur Förderrichtung angeordnet und mit den einzelnen Ausnehmungen (23, 123) verbunden sind, und die freigesetzten Akkumulationen (32, 32', 132) befördert werden, während sie einzeln angesaugt werden.

4. Verfahren zur Herstellung des absorbierenden Elements nach Anspruch 1 oder 3, wobei die Fördereinrichtung durch eine Übertragungswalze (5, 105) und ein an der äußern Umfangsoberfläche der Übertragungswalze angebrachtes Netzband (7, 107) definiert ist, oder ein Vakuumfördermittel (8, 8A, 108).

5. Gerät zur Herstellung eines absorbierenden Elements das aufweist: eine Drehtrommel (2, 102) mit Ausnehmungen (23, 123) an ihrer äußeren Umfangsoberfläche, die mehrere feine Poren an entsprechenden Böden aufweisen, und das ausgebildet ist, um Material für das absorbierende Element zu akkumulieren, das aus einer Materialzufuhr-Zone in den jeweiligen Ausnehmungen (23, 123) zugeführt wird, um Akkumulationen (32, 32', 132) aus den Ausnehmungen (23, 123) freizusetzen, und um diese auf eine Fördereinrichtung zu übertragen so dass die Akkumulationen (32, 32', 132) befördert werden können, während sie auf der Fördereinrichtung gehalten werden, wobei:
mehrere Ausnehmungen (23, 123) so angebracht ist, dass sie sowohl in einer Umfangsrichtung als auch in einer Breitenrichtung der Drehtrommel (2, 102) voneinander beabstandet sind, wobei die Fördereinrichtung mit Saugporen (53, 53') ausgestattet ist, die so auf der Fördereinrichtung in einer Richtung rechtwinklig zu deren Förderrichtung angebracht und einzeln mit mehreren Ausnehmungen (23, 123) verbunden sind, dass diese Saugporen (53, 53') die Akkumulationen (32, 32', 132) einzeln in die Ausnehmungen saugen können.

6. Gerät zur Herstellung des absorbierenden Elements nach Anspruch 5, wobei jede der Saugporen (53, 53') eine Dimension in eine Richtung rechtwinklig zur Förderrichtung hat, die gleich groß ist wie, oder kleiner als eine Dimension jeder der Ausnehmungen (23, 123) in der Breitenrichtung der Drehtrommel (2, 102), und die eine Dimension in der Förderrichtung hat, die gleich groß ist wie, oder kleiner als eine Dimension jeder der Ausnehmungen (23, 123) in der Umfangsrichtung der Drehtrommel (2, 102).

7. Gerät zur Herstellung des absorbierenden Elements nach Anspruch 5 oder 6, wobei eine Windschutzplatte (9, 9'), die in der Nähe der Fördereinrichtung angebracht ist, dazu dient, den Wind zu verhindern oder zu verringern, der ansonsten in einen Bereich fließen würde, der die in einer Richtung rechtwinklig zur Förderrichtung angebrachten Saugporen einschließt.

8. Gerät zur Herstellung des absorbierenden Elements nach Anspruch 5, 6, oder 7, wobei mehrere Saugporenreihen, die jeweils mehrere nacheinander in der Förderrichtung der vorgenannten Fördereinrichtung angebrachte Saugporen (53, 53') aufweisen, in der Richtung rechtwinklig zur Förderrichtung ausgebildet sind, und eine die Reihen trennende Platte zwischen jedem Paar benachbarter Saugporenreihen vorgesehen ist.

9. Gerät zur Herstellung des absorbierenden Elements nach einem der Ansprüche 5 bis 8, wobei die Fördereinrichtung durch eine Übertragungswalze (5, 105) und ein an der äußeren Umfangsoberfläche der Übertragungswalze (5, 105) angebrachtes Netzband definiert ist, oder ein Vakuumfördermittel (8, 8A, 108).

10. Gerät zur Herstellung des absorbierenden Elements nach Anspruch 5, wobei die Fördereinrichtung durch eine Übertragungswalze (5, 105) und ein an der äußern Umfangsoberfläche der Übertragungswalze (5, 105) angebrachtes Netzband (7, 107) definiert ist, und eine Vakuumbox (6, 106) zwischen der Materialzufuhr-Zone und der Übertragungswalze (5, 105) angeordnet ist, so dass die Vakuumbox (6, 106) die Akkumulationen (32, 32', 132) in den Ausnehmungen (23, 123) der der Drehtrommel (2, 102) gegenüberliegenden Seite saugt.

11. Gerät zur Herstellung des absorbierenden Elements nach Anspruch 10, wobei das Netzband (7, 107) geeignet ist, kontinuierlich zwischen der Vakuumbox (6, 106) und der Übertragungswalze (5, 105) zu laufen.

## Revendications

1. Méthode pour fabriquer un élément absorbant comprenant une étape d'accumulation comprenant l'accumulation de matériau pour l'élément absorbant porté par l'écoulement d'air dans des évidements (23, 123) formés sur une surface périphérique extérieure d'un tambour rotatif (2, 102) par aspiration, et une étape de transport comprenant le transfert d'accumulations (32, 32', 132) dans les évidements (23, 123) sur un moyen de transport et le transport des accumulations alors que les accumulations (32, 32', 132) sont retenues sur le moyen de transport, dans laquelle :
dans l'étape d'accumulation, le matériau pour l'élément absorbant est accumulé dans une pluralité des évidements (23, 123) formés sur le tambour rotatif (2, 102) afin d'être agencés dans un sens de la largeur du tambour rotatif (2, 102) ;
et dans l'étape de transport, les accumulations (32, 32', 132) sont libérées des évidements (23, 123) sur le moyen de transport sous une force d'aspiration fournie à partir du moyen de transport et les accumulations libérées (32, 32', 132) sont transportées tout en étant aspirées, dans laquelle la force d'aspiration est fournie à partir d'une pluralité de pores d'aspiration (53, 53') agencés dans le moyen de transport dans un sens orthogonal à son sens de transport afin d'être associés aux évidements respectifs (23, 123) et les accumulations (32, 32', 132) ayant été libérées sont transportées tout en étant individuellement aspirées.

2. Méthode pour fabriquer l'élément absorbant selon la revendication 1, comprenant en outre une étape d'adhésion des accumulations (32, 32', 132) dans les évidements (23, 123) à un élément en feuille perméable à l'air prévu sur les évidements (23, 123) et le transport des accumulations (32, 32', 132) dans cet état avant que les accumulations (32, 32', 132) soient transférées au moyen de transport.

3. Méthode pour fabriquer l'élément absorbant selon la revendication 1, dans laquelle :
dans l'étape d'accumulation, le matériau pour l'élément absorbant est accumulé dans une pluralité des évidements (23, 123) formés sur le tambour rotatif (2, 102) afin d'être agencés dans le sens circonférentiel et dans le sens de la largeur, respectivement ; et
dans l'étape de transport, les accumulations (32, 32', 132) sont libérées des évidements (23, 123) sur le moyen de transport susmentionné sous la force d'aspiration fournie à partir d'une pluralité de pores d'aspiration (53, 53') formés dans le moyen de transport afin d'être agencés dans un sens de transport et dans un sens orthogonal à ce sens de transport et d'être associés aux évidements individuels (23, 123) et les accumulations (32, 32', 132) ayant été libérées sont transportées tout en étant individuellement aspirées.

4. Méthode pour fabriquer l'élément absorbant selon la revendication 1 ou 3, dans laquelle le moyen de transport est défini par un rouleau de transfert (5, 105) et une courroie maillée (7, 107) prévue sur une surface périphérique extérieure du rouleau de transfert, ou un transporteur à vide (8, 8A, 108).

5. Appareil pour fabriquer un élément absorbant comprenant un tambour rotatif (2, 102) comportant des évidements (23, 123) sur sa surface périphérique extérieure qui comportent une pluralité de pores fins sur des fonds respectifs, et étant configuré pour accumuler un matériau pour l'élément absorbant fourni à partir d'une zone d'alimentation en matériau dans les évidements respectifs (23,123), pour libérer des accumulations (32, 32', 132) à partir des évidements (23, 123) et pour les transférer sur le moyen de transport pour que les accumulations (32, 32', 132) puissent être transportées tout en étant retenues sur le moyen de transport, dans lequel :
une pluralité des évidements (23, 123) sont agencés afin d'être espacés les uns des autres dans un sens circonférentiel ainsi que dans un sens de la largeur du tambour rotatif (2, 102), dans lequel le moyen de transport est pourvu de pores d'aspiration (53, 53') agencés dans le moyen de transport dans un sens orthogonal à son sens de transport et individuellement associés à une pluralité des évidements (23, 123) pour que ces pores d'aspiration (53, 53') puissent individuellement aspirer les accumulations (32, 32', 132) dans les évidements.

6. Appareil pour fabriquer l'élément absorbant selon la revendication 5, dans lequel chacun des pores d'aspiration (53, 53') possède une dimension dans un sens orthogonal au sens de transport égale ou inférieure à une dimension de chacun des évidements (23, 123) dans le sens de la largeur du tambour rotatif (2, 102) et possède une dimension dans le sens de transport égale ou inférieure à une dimension de chacun des évidements (23, 123) dans le sens circonférentiel du tambour rotatif (2, 102).

7. Appareil pour fabriquer l'élément absorbant selon la revendication 5 ou 6, dans lequel une plaque pare-vent (9, 9') installée dans le voisinage du moyen de transport sert à empêcher ou réduire le vent qui s'écoulerait autrement dans une région comprenant les pores d'aspiration dans un sens orthogonal au sens de transport.

8. Appareil pour fabriquer l'élément absorbant selon les revendications 5, 6, ou 7, dans lequel une pluralité de colonnes de pores d'aspiration comprenant chacune une pluralité des pores d'aspiration (53, 53') agencés en série dans le sens de transport du moyen de transport susmentionné sont formées dans le sens orthogonal au sens de transport, et une plaque de séparation inter-colonne est prévue entre chaque paire de colonnes de pores d'aspiration adjacentes.

9. Appareil pour fabriquer l'élément absorbant selon une quelconque des revendications 5 à 8, dans lequel le moyen de transport est défini par un rouleau de transfert (5, 105) et une courroie maillée prévue sur une surface périphérique extérieure du rouleau de transfert (5, 105), ou un transporteur à vide (8, 8A, 108).

10. Appareil pour fabriquer l'élément absorbant selon la revendication 5, dans lequel le moyen de transport est défini par un rouleau de transfert (5, 105) et une courroie maillée (7, 107) prévue sur la surface périphérique extérieure du rouleau de transfert (5, 105), et une caisse aspirante (6, 106) est située entre la zone d'alimentation en matériau et le rouleau de transfert (5, 105) pour que la caisse aspirante (6, 106) aspire les accumulations (32, 32', 132) dans les évidements (23, 123) à partir du côté opposé au côté du tambour rotatif (2, 102).

11. Appareil pour fabriquer l'élément absorbant selon la revendication 10, dans lequel la courroie maillée (7, 107) est adaptée pour passer entre la caisse aspirante (6, 106) et le rouleau de transfert (5, 105) de manière continue.
